# FASCICULE DE BREVET EUROPEEN

(11) **EP 1 094 777 B1**
(45) Date de publication et mention de la délivrance du brevet: **13.09.2006**
(21) Numéro de dépôt: 98936470.8
(22) Date de dépôt: 08.07.1998
(51) Int. Cl.: A61F 15/00, B65D 81/32

(54) **APPLICATEUR A MULTI-COMPARTIMENT A USAGE UNIQUE**
EINWEG-MEHRKAMMERAPPLIKATOR
DISPOSABLE APPLICATOR WITH MULTIPLE CELLS

(43) Date de publication de la demande: 02.05.2001
(73) Titulaire: Fleury, Lydie, 95380 Puisseux (FR); Tir Groupe, 92802 Puteaux (FR); Verpackungs Service GmbH, 76356 Weingarten (DE)
(72) Inventeur: Fleury, Lydie, 95380 Puisseux (FR); Tir Groupe, 92802 Puteaux (FR); Verpackungs Service GmbH, 76356 Weingarten (DE)
(74) Mandataire: Ahner, Francis
(86) Numéro de dépôt international: PCT/FR1998/001463
(87) Numéro de publication internationale: WO 2000/002513

(56) Documents cités:
- EP-A- 0 737 463
- US-A- 3 826 259
- US-A- 5 558 874
- US-A- 5 681 574

## Description

Ce dispositif à caractère jetable et usage unique contenant des éléments de soin et à caractère médical, pour cet exemple qui n'est en aucun cas limitatif en ce qui concerne les substances, composants, matériaux employés et assemblés, ainsi que pour les dimensions et forme présentées, garantit une non-contamination et une stérilisation totale et durable des divers éléments conditionnés et assemblés.

Son utilisation et application à caractère innovant par rapport aux antériorités d'ensemble formant un nécessaire pelable ou sécable ou extractible, sont confirmées par le principe de fonctionnement par pression, éclatement et découvrement. Ces trois principes permettent l'utilisation et l'application de ce dispositif sans jamais désolidariser les éléments contenus de l'élément contenant, garantissant ainsi une parfaite stérilité de l'ensemble et une non contamination des éléments, applicable avant et pendant l'utilisation. De plus, compte tenu qu'aucun élément ne peut être perdu ou désolidarisé de l'ensemble, cela assure une protection de l'environnement.

Cette invention présentée pour une application de soin à caractère médical peut être de même façon applicable dans le domaine cosmétique, alimentaire, nettoyage, entretiens divers, etc...

Avec les éléments adéquats composant l'ensemble du dispositif en fonction des domaines destinés à son application.

### DESCRIPTION

Cette invention présente un dispositif regroupant plusieurs éléments et produits conditionnés et assemblés de façon totalement hermétique et sans risque pour l'utilisateur quels que soient le domaine, les conditions d'application, l'âge de l'utilisateur et ses connaissances en ces domaines.

A aucun moment, avant ou pendant l'application, l'utilisateur n'est au contact et cela pour la totalité des produits (liquide, crème, pâte, tampons d'application, pansements adhésifs de protection), pour ce cas de figure dans le domaine du soin et de l'hygiène corporelle, mais également dans les autres domaines d'application.

Des antériorités existent concernant divers emballages regroupant des éléments pour interventions diverses:
-US-A-2377117
-FR-A-2 255 224
- DE - C - 1 087 758
-FR-A- 1 111 282
- EP-O-737 463 A1

D'autres documents décrivent des dispositifs de premier soin comportant deux réservoirs pouvant communiquer par un canal sur une valve étroite (EP-0 737 463, US-5,558,874, US-5,681,574).

Différentes particularités et avantages conséquents, différenciant cette invention des techniques antérieures déjà connues :

Association de trois éléments indissociables et ne faisant qu'un pour une application.

Totale herméticité, stérilité et non contaminable du fait que le contenant soit monobloc et ne se constitue pas de pièces rapportées

Communication des éléments liquides et solides (sans canal de distribution pouvant comporter des défauts de fonctionnement par obstruction) mais par simple pression.

Assurance du maintien solidaire de tous les éléments du dispositif avant, pendant et après utilisation, grâce à sa structure monobloc sans éléments extractibles, ni pelables, garantissant ainsi sa stérilité avant et pendant l'utilisation et la protection de l'environnement par l'élimination du risque d'égarement d'un des éléments du dispositif.

Compte tenu de sa conception simplifiée ce qui en découle, une réalisation offrant toutes les garanties de sécurité et de stérilité de façon industrielle, met à la portée financière de tous les utilisateurs quels que soient leur degré d'affranchissement ou leur âge pour le domaine de l'application.

Dispositif à usage unique et jetable de premier soin représentant un ensemble ne faisant qu'un, caractérisé par sa simplicité d'utilisation, l'assurance de non-contamination avant, pendant son utilisation et le non-risque de contamination et de souillure de l'utilisateur ou par l'utilisateur, se compose d'un élément thermoformé à deux empreintes faisant office de réceptacle, respectivement pour l'élément fluide et l'élément sec, préservé de toute contamination par un thermocellage aluminium, plastique ou tout autre matériau utilisé dans le monde industriel garantissant une totale herméticité.

La relation de l'élément liquide par rapport à l'élément sec se faisant par simple pression du réceptacle liquide, provoquant systématiquement une désolidarisation partielle du thermocellage et une imbibation de l'élément sec tout en garantissant une totale herméticité de l'ensemble.

Autre élément important de l'invention, caractérisé par la disposition et de manière indissociable sur le thermocellable rendant solidaire et monobloc l'ensemble du dispositif, un étui (ou pochette) renfermant un adhésif de protection dans le cas présent pour une plaie après désinfection, celui-ci étant préhensible non pas par pelage de l'étui, mais par déchirure centrale de l'étui, ce qui a pour avantage de ne jamais être au contact de l'adhésif au moment de sa préhension et de son application.

Une réalisation schématique du dispositif caractérisant l'invention est représentée à titre d'exemple non limitatif quant aux formes et dimensions.
Fig 1 - Vue de face en coupe
Fi g 2 - Vue de dessous
Fi g 3 - Vue de dessus
Fig 4 . Vue éclatée
Fig 5 - Vue fonctionnelle

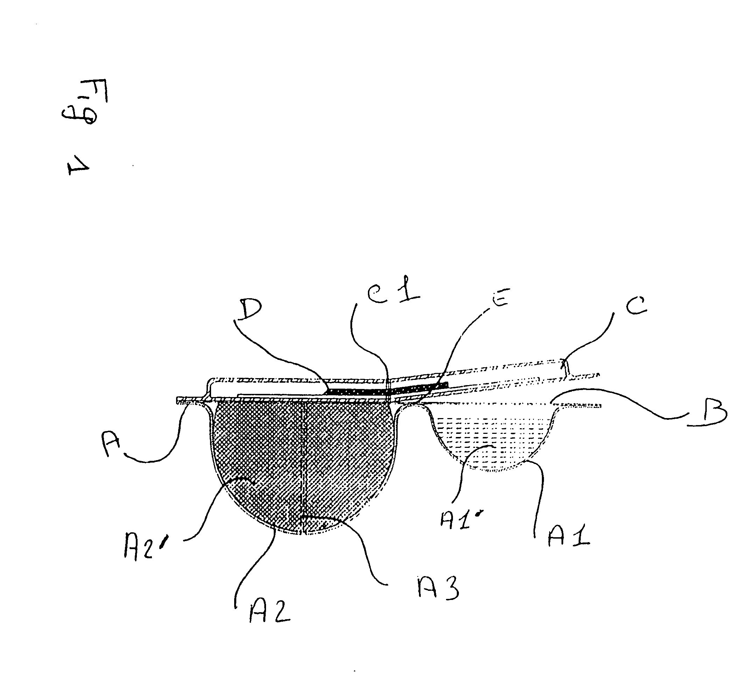

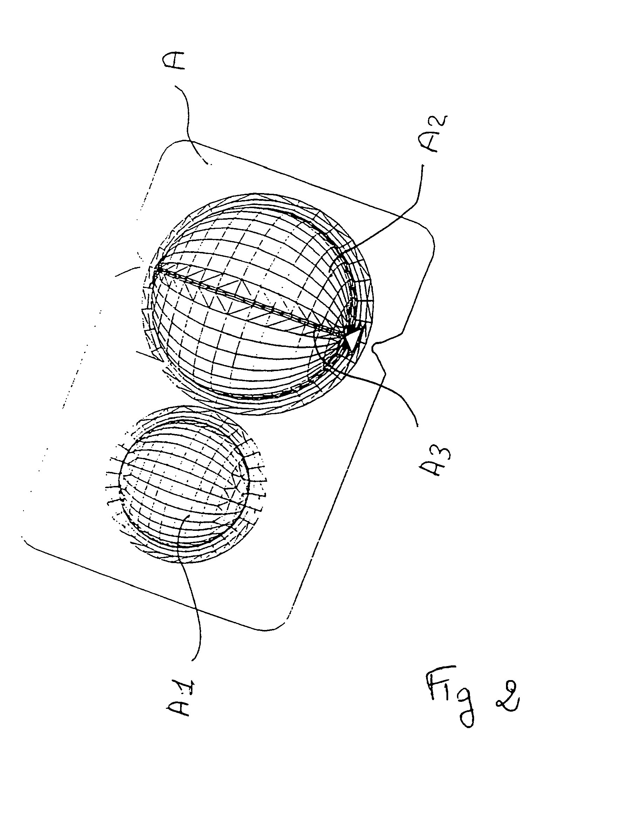

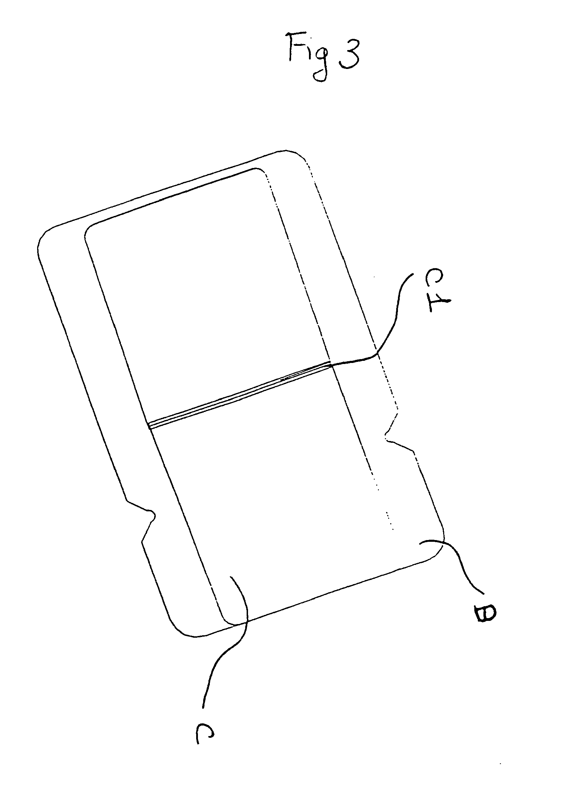

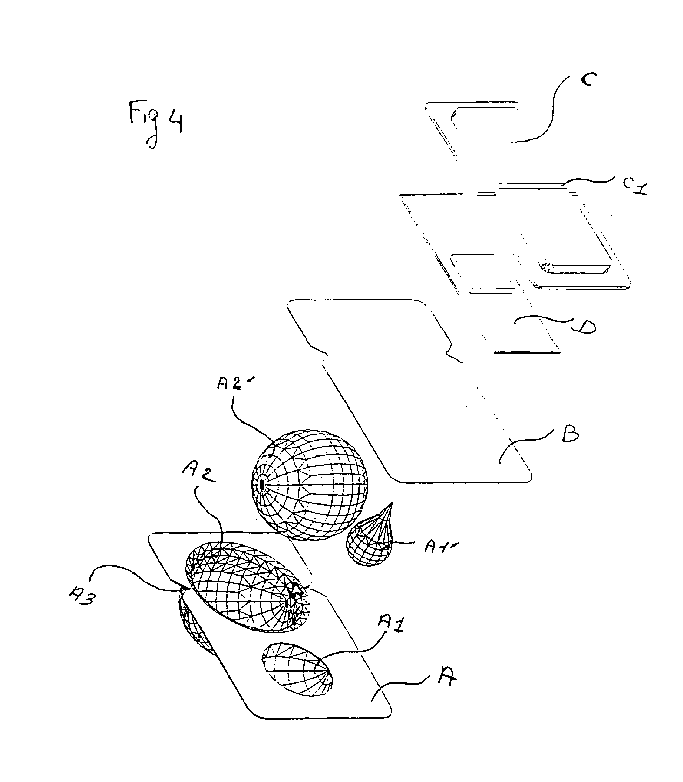

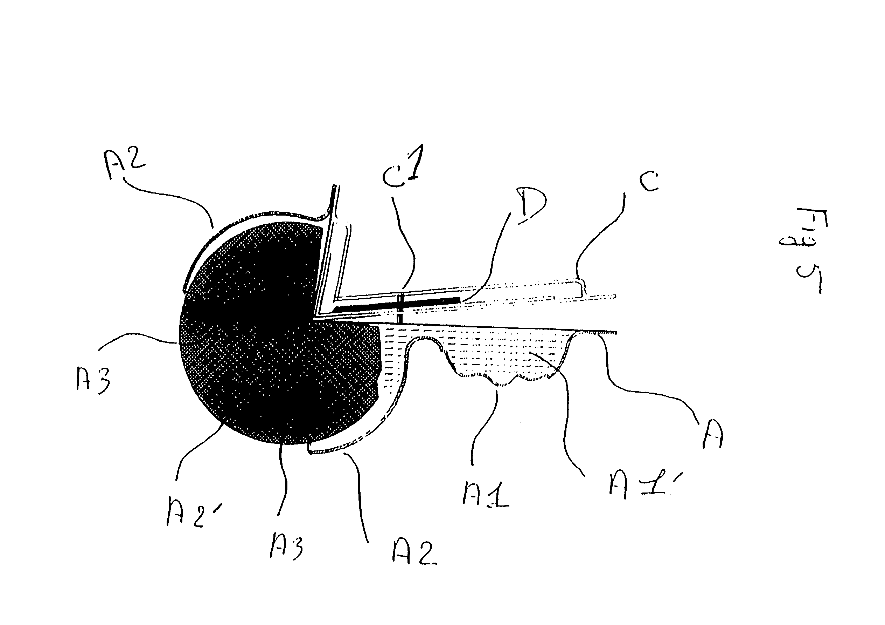

Descriptif d'utilisation et d'application, ces informations sont données pour un dispositif de soin corporel, sans être limitatif à tout autre type d'application.

Illustration par les dessins représentant une plaque thermoformée A à deux empreintes A 1 A2 respectivement prévues pour A1 le conditionnement de l'élément liquide, crémeux ou pâteux A l'et pour A2 à l'élément sec, tampon, compresse ou tout autre élément à fibre synthétique ou naturelle à pouvoir d'absorption et de restitution A2', l'empreinte A2 caractérisée par le fait qu'elle comporte une pré-découpe transversale A3 permettant le découvrement de l'élément sec après son imbibation pour son application.

Le maintien des éléments secs et liquides dans leurs empreintes respectives est assuré et de façon totalement hermétique et stérile par un film thermoscellable B recouvrant de manière indissociable la face opposée des empreintes réceptacles des éléments liquides et secs.

Ce film B étant lui-même doté d'une pochette C déchirable en son centre C1 et renfermant un adhésif de protection cutanée D dans ce cas de figure.

### Utilisation du dispositif

On exerce une simple pression sur l'empreinte A1 contenant l'élément fluide A1', ce qui a pour effet de désolidariser à l'endroit le plus faible le film thermoscellable B de la plaque A permettant ainsi la communication de l'élément fluide A l'avec l'élément sec, cet endroit le plus faible E est situé entre les empreintes A1 et A2. Une fois l'applicateur A2' imprégné, il suffit de procéder au pliage de l'empreinte A2 à 180°, pliage et découvrement de l'applicateur imprégné favorisé par la pré-découpe transversale A3.

Ce procédé a pour avantage certain de ne jamais toucher l'applicateur imprégné A2' et de garantir son maintien dans l'empreinte A2 au moment de l'application, l'ensemble restant solidaire et indissociable, garantissant ainsi une non contamination et une préservation de l'environnement.

Après application de l'élément fluide, crémeux ou pâteux A1' désinfectant, asséchant ou assainissant par l'intermédiaire de l'applicateur A2' compresse, tampon, élément absorbant et restituant en matière ou fibre synthétique ou naturelle, on procède à la préhension de l'adhésif de protection cutanée D par déchirement central C1) de la pochette C, ce qui a pour avantage de maintenir et d'appliquer l'adhésif D avec la partie restante de la pochette (C) (ou demi-pochette C) offrant la garantie de ne jamais toucher et contaminer l'adhésif protecteur cutané au moment de son application.

## Revendications

1. Dispositif de premiers soins à usage unique, jetable, garantissant une stérilité et non contamination durable des éléments et substances assemblées, **caractérisé en ce qu'**il comprend un contenant unique et monobloc (A) comprenant deux empreintes réceptacles (A1, A2), hermétiquement obturé par un film (B), l'une des empreintes réceptacles (A1) étant prévue pour le conditionnement d'un élément liquide, crémeux ou pâteux (A1') et l'autre empreinte réceptacle (A2) étant prévue pour le conditionnement d'un élément sec tampon, compresse ou tout autre élément en matière ou à fibre synthétique ou naturelle, à pouvoir d'absorption et de restitution (A2'), une zone fragilisée (E) de l'obturation hermétique du contenant (A) par le thermoscellage d'un film (B) étant prévue entre les deux éléments (A1') et (A2') pour permettre, par simple pression sur l'empreinte réceptacle (A1) contenant l'élément liquide (A1'), la désolidarisation au moins partielle du film (B) d'avec le contenant (A ) dans cette zone fragilisée (E) et ainsi la communication de l'élément liquide (A1') avec l'élément sec (A2') tout en maintenant le dispositif totalement hermétique.

2. Dispositif selon la revendication 1, **caractérisé en ce qu'**il comporte en outre une pré-découpe (A3) située transversalement sur l'empreinte réceptacle (A2) pour permettre, après désolidarisation au moins partielle du film (B) d'avec le contenant (A ) dans la zone fragilisée (E) et imbibation de l'élément sec (A2') par l'élément liquide (A1') dans le dispositif totalement hermétique, de découvrir l'élément sec (A2') imbibé par l'élément liquide (A1'), par simple pliage du dispositif tout en maintenant l'élément sec (A2') solidaire de son empreinte réceptacle (A2) au moment de l'utilisation, sans risque de contamination et de déperdition.

3. Dispositif selon la revendication 2, **caractérisé en ce que** la pré-découpe (A3) permet un pliage du dispositif selon un angle d'environ 180°

4. Dispositif selon l'une quelconque des revendications 1 à 3, **caractérisé en ce qu'**il comporte en outre une pochette (C), disposée de façon indissociable sur sa demi-longueur avec le film (B), présentant en son centre une zone déchirable (C1) et renfermant un adhésif de protection cutanée (D), pour permettre, par déchirement de la zone déchirable (C1), d'appréhender l'adhésif (D) de façon non contaminante par la partie déchirée de la pochette (C) et ainsi appliquer l'adhésif de façon non contaminante par sa partie libérée de la pochette (C) déchirée.

## Claims

1. A disposable single-use first-aid device guaranteeing long-lasting sterility and non-contamination of assembled-together elements and substances, **characterized in that** it comprises a single one-piece container (A) having two receptacle blisters (A1, A2) hermetically closed by a film (B), one of the receptacle blisters (A1) being for packaging a liquid, cream, or paste element (A1') and the other receptacle blister (A2) being for packaging a dry element (A2'), pad, compress, or any other element of synthetic or natural fibber or material having absorption and restitution power, a zone of weakness (E) being provided in the hermetic closure of the container (A) by heat-sealing a film (B) between the two elements (A1' and A2') to make it possible, merely by squeezing the receptacle blister (A1) containing the liquid element (A1'), to separate at least part of the film (B) from the container (A) in this zone of weakness (E), thereby putting the liquid element (A1') into communication with the dry element (A2') while keeping the device totally hermetic.

2. A device according to claim 1, **characterized in that** it further comprises a line of weakness (A3) situated transversely on the receptacle blister (A2) to make it possible, after at least part of the film (B) has been separated from the container (A) in the zone of weakness (E) and the dry element (A2') has been soaked by the liquid element (A1') in the totally hermetic device, to uncover the dry element (A2') soaked by the liquid element (A1') merely by folding the device while keeping the dry element (A2') integral with its receptacle blister (A2) at the moment of use without any risk of it being contaminated or lost.

3. A device according to claim 2, **characterized in that** the line of weakness (A3) enables the device to be folded through an angle of about 180°.

4. A device according to any one of claims 1 to 3, **characterized in that** it further comprises a pocket (C) disposed over half its length in indissociable manner with the film (B), the pocket having a tear zone (C1) in its center and containing a sticking-plaster (D) for protecting the skin, thereby making it possible by tearing the tear zone (C1) to take hold of the sticking-plaster (D) in non-contaminating manner by holding the torn portion of the pocket (C) and thus applying the sticking-plaster in non-contaminating manner via its portion that has been released by the torn pocket (C).

## Patentansprüche

1. Wegwerfbare Erste-Hilfe-Vorrichtung zur einmaligen Verwendung, die eine dauerhafte Sterilität und Nichtverunreinigung der zusammengestellten Elemente und Substanzen garantiert, **dadurch gekennzeichnet, dass** sie einen einzelnen und einstückigen Behälter (A) umfasst, der zwei Aufnahmevertiefungen (A1, A2) umfasst, die von einer Folie (B) verschlossen sind, wobei die eine der Aufnahmevertiefungen (A1) für das Verpacken eines flüssigen, cremigen oder pastösen Elements (A1') vorgesehen ist und die andere Aufnahmevertiefung (A2) für das Verpacken eines trockenen Bauschelements, Kompressenelements oder jedes anderen Elements aus synthetischem/r oder natürlichern/r Material oder Faser mit Absorptions- und Wiedergabevermögen (A2') vorgesehen ist, eine Aufbrechstelle (E) des hermetischen Verschlusses des Behälters (A) durch das Heißsiegeln einer Folie (B), die zwischen den beiden Elementen (A1') und (A2') vorgesehen ist, um durch einfachen Druck auf die das flüssige Element (A1') enthaltende Aufnahmevertiefung (A1) die wenigstens teilweise Trennung der Folie (B) vom Behälter (A) an dieser Aufbrechstelle (E) und somit die Verbindung des flüssigen Elemente (A1') mit dem trockenen Element (A2') zu erlauben, während gleichzeitig die Vorrichtung völlig hermetisch bleibt.

2. Vorrichtung nach Anspruch 1, **dadurch gekennzeichnet, dass** sie ferner eine Vorstanzung (A3) umfasst, die quer zur Aufnahmevertiefung (A2) liegt, um nach der wenigstens teilweisen Trennung der Folie (B) vom Behälter (A) an der Aufbrechstelle (E) und Tränkung des trockenen Elements (A2') durch das flüssige Element (A1') in der völlig hermetischen Vorrichtung, das vom flüssigen Element (A1') getränkte trockene Element (A2') durch einfaches Knicken der Vorrichtung freilegen zu können, während gleichzeitig das trockene Element (A2') zum Verwendungszeitpunkt mit seiner Aufnahmevertiefung (A2) verbunden bleibt, ohne Verunreinigungs- und Verlierrisiko.

3. Vorrichtung nach Anspruch 2, **dadurch gekennzeichnet, dass** die Vorstanzung (A3) ein Knicken der Vorrichtung um einen Winkel von ungefähr 180° erlaubt.

4. vorrichtung nach einem der Ansprüche 1 bis 3. , **dadurch gekennzeichnet, dass** sie ferner eine Hülle (C) umfasst, die über ihre Halblänge untrennbar mit der Folie (B) vorliegt, wobei sie in ihrer Mitte eine aufreißbare Stelle (C1) aufweist und einen Hautschutzhaftkleber (D) einschließt, um durch Aufreißen der aufreißbaren Stelle (C1), den Kleber (D) auf nicht verunreinigende weise über den zerrissenen Abschnitt der Hülle (C) ergreifen und so den Kleber auf nichtverunreinigende Weise über seinen von der zerrissenen Hülle freigegebenen Abschnitt aufbringen zu können.
